Europäisches Patentamt

European Patent Office

Office européen des brevets

(19)

(11) Veröffentlichungsnummer : **0 183 992**
**B1**

(12) **EUROPÄISCHE PATENTSCHRIFT**

(45) Veröffentlichungstag der Patentschrift :
07.06.89

(21) Anmeldenummer : 85113862.8

(22) Anmeldetag : 31.10.85

(51) Int. Cl.⁴ : **A 61 F 15/00, A 61 F 13/00**

(54) Schutzhülle zum flüssigkeitsdichten Abdecken von Körperteilen.

(30) Priorität : 07.12.84 DE 3444626

(43) Veröffentlichungstag der Anmeldung :
11.06.86 Patentblatt 86/24

(45) Bekanntmachung des Hinweises auf die Patenterteilung : 07.06.89 Patentblatt 89/23

(84) Benannte Vertragsstaaten :
AT BE CH DE FR GB IT LI LU NL SE

(56) Entgegenhaltungen :
EP--A-- 0 039 184
DE--A-- 3 030 601
DE--A-- 3 304 086
GB--A-- 1 567 179
US--A-- 3 329 143
US--A-- 4 363 317

(73) Patentinhaber : HORMON-CHEMIE GMBH
Freisinger Landstrasse 74
D-8000 München 45 (DE)
DE
CHEMIE HOLDING AKTIENGESELLSCHAFT
Postfach 296 St. Peter-Strasse 25
A-4021 Linz (AT)
BE CH FR GB IT LI LU NL SE AT

(72) Erfinder : Wiesenthal, Peter, Dr.
Niedersachsenstrasse 51
D-2090 Winsen/Luhe (DE)
Erfinder : Ille, Beatus
Niedersachsenstrasse 44
D-2090 Winsen/Luhe (DE)

(74) Vertreter : Meier, Friedrich, Dipl.-Ing.
Philips Patentverwaltung GmbH Wendenstrasse 35
Postfach 10 51 49
D-2000 Hamburg 1 (DE)

EP 0 183 992 B1

## Beschreibung

Sind an Körperteilen, z. B. Arme oder Beine, Verbände angelegt, so besteht insbesondere dann, wenn diese Verbände über einen längeren Zeitraum, wie z. B. Gipsverbände, nicht abgenommen werden können, das dringende Bedürfnis, diese Verbände z. B. beim Duschen gegen das Eindringen von Wasser abzudecken. In vielen Fällen haben sich Patienten dadurch geholfen, daß sie sackartige Kunststoffhüllen über die Körperteile gestülpt und dann z. B. mit Hilfe von Schnüren, Gummiringen oder Bändern am Körperteil befestigt haben. Selbstverständlich ergeben solche Abdeckungen keinen wasserdichten Verschluß, denn die zusammengefalteten Kunststoffolien bilden in jedem Falle feine Kanäle, durch die das Wasser z. B. beim Duschen eindringen kann.

Aus der US-A-PS 4.363.317 ist eine wasserdichte Schutzhülle zur Abdeckung von Gipsverbänden bekannt, bei der im Bereich der Schlupföffnung ein elastisches Band mit einander überlappenden Enden befestigt ist. Die überlappenden Enden sind mit Haftteilen versehen, so daß sie übereinanderliegend das abzudeckende Körperteil umschließen. Diese bekannte Ausführungsform hat den Nachteil, daß das Band praktisch auf der ganzen Länge mit Ausnahme des überlappenden Teiles am oberen Rand im Bereich der Schlupföffnung befestigt ist. Damit läßt sich die Abdeckung nur sehr schwer unter Verwendung von eingearbeiteten Kerben an unterschiedliche Größen der abzudeckenden Körperteile anpassen. Ferner hat diese bekannte Ausführungsform den Nachteil, daß die Dichtung ausschließlich durch die Elastizität des Bandes erfolgt, daß also die Körperteile sehr stark eingeschnürt werden müssen, um eine gewisse Abdichtung zu erreichen.

Aus der DE-A-3 304 086 ist eine Abdecktasche bekannt, durch die Extremitäten z. B. bei chirurgischen Eingriffen abgedeckt werden können. Die Tasche besteht aus einem Ober- und einem Unterteil sowie eingearbeiteten Falten. Das Unterteil ist zudem mit angeschnittenen, seitlich überstehenden Bändern versehen, die nach Anlegen der Tasche selbstklebend um das abzudeckende Körperteil gelegt werden können. Diese bekannte Ausführungsform hat den wesentlichen Nachteil, daß die abstehenden Bandenden über einen großen Bereich des Taschenrandes mit dieser verbunden sind, so daß die Tasche nur schwer an unterschiedliche Größen angepaßt werden kann. Die Tasche hat ferner den Nachteil, daß sie von einem Patienten allein nicht angelegt werden kann, weil beim Anlegen die Tasche gehalten, dann das eine Bandende herumgelegt und dann erst das andere Bandende überlappend befestigt werden kann. Im übrigen ergibt diese Tasche keine wasserdichte Abdeckung.

Nimmt man eine normale Kunststoffhülle und benutzt zum Abdichten der Folie am Körper Klebstreifen, wie sie für Wundverbände verwendet werden, dann wird eine verhältnismäßig gute Abdichtung gegen das Eindringen von Wasser erreicht. Das Anlegen einer solchen Schutzhülle ist aber zumindest an den Armen außerordentlich schwierig, weil die Schutzhülle festgehalten und gleichzeitig der Klebstreifen aufgebracht werden muß. Im übrigen sind diese Art Klebstreifen für eine Abdichtung nicht vorgesehen und sind deshalb zum Teil nicht wasserdicht, kleben an feuchten Körperstellen schlecht und sind an einzelnen Körperteilen nur wieder unter Schmerzen zu entfernen, weil ihre Haftkraft, z. B. an Haaren, außerordentlich groß ist.

Der Erfindung liegt die Aufgabe zugrunde, eine Schutzhülle zu schaffen, die speziell zum flüssigkeitsdichten Abdecken von Körperteilen geeignet ist und dabei von einer sackartigen Kunststoffolie, z. B. aus Polyethylen, mit einer Schlupföffnung Gebrauch macht, an der ein bandförmiger elastischer Streifen so befestigt ist, daß er gegen die Schlupfrichtung über den Rand der Hülle übersteht und mit der Innenseite eines frei abstehenden Endes auf seiner Außenseite haftet. Die Schutzhülle sollte unter grober Anpassung an die Konturen der abzudeckenden Körperteile, z. B. einem Fuß, leicht auch mit einer Hand flüssigkeitsdicht zu befestigen sein und einen guten Abschluß ergeben.

Gemäß der Erfindung wird dies dadurch erreicht, daß der bandförmige, auf der Innenseite mit Klebstoff belegte Dichtstreifen aus zähelastischem Material mit dem einen Ende nur auf einem Teil des Umfanges der Schlupföffnung befestigt und mit dem anderen Ende einen einseitig frei abstehenden Dichtstreifen solcher Länge und solcher Oberfläche bildet, daß der Dichtstreifen nach mehr als einmaligem Umschließen des abzudeckenden Körperteiles unter Einschluß des Randes der Hülle aufeinanderklebend überlappt und daß im Bereich des Schlupfrandes an der Innenseite der Hülle ein Stück Klebstreifen als Haftstelle befestigt ist.

Vorteilhaft ist es, den Dichtstreifen aus einem hautfreundlichen Material zu machen, das flüssigkeitsdicht ist und möglichst auch an feuchten Körperteilen gut, aber nicht zu fest haftet. Beim Umschließen eines Körperteiles mit dem Dichtstreifen ist die Haftfestigkeit am Körper selbst nicht so wichtig wie der dichte Abschluß und die gute Haftung im Bereich der Überlappung. Zum Festhalten der Hülle, z. B. bei einhändiger Bedienung, ist an der Innenseite der Hülle ein Stück Klebstreifen als Haftstelle befestigt, die ebenso wie der Dichtstreifen im Bereich aufgetragener Klebeschichten mit einer Schutzfolie abgedeckt ist, die kurz vor der Anwendung abgezogen wird. Durch den an der Innenseite der Hülle angebrachten Klebstreifen wird die Schutzhülle am Körperteil festgelegt, so daß dann der Dichtstreifen straff um das Körperteil unter Einschluß der gefalteten oberen Kante der Schutzhülle gelegt werden kann.

In besonderen Fällen kann der obere Rand der Schutzhülle zusätzlich mit einem weichelastischen Wulst versehen werden, der z. B. aus Moosgummi besteht und am Dichtstreifen kurz oberhalb des Randes der Schutzhülle an der Innenseite aufgebracht ist. Damit könnte eine Flüssigkeitsabdichtung auch mit einem Dichtstreifen erzielt werden, der an den Körperteilen selbst nur sehr wenig oder überhaupt nicht haftet und nur im Überlappungsbereich aufeinanderklebt.

Anhand der Zeichnung sei ein Ausführungsbeispiel der Erfindung beschrieben.

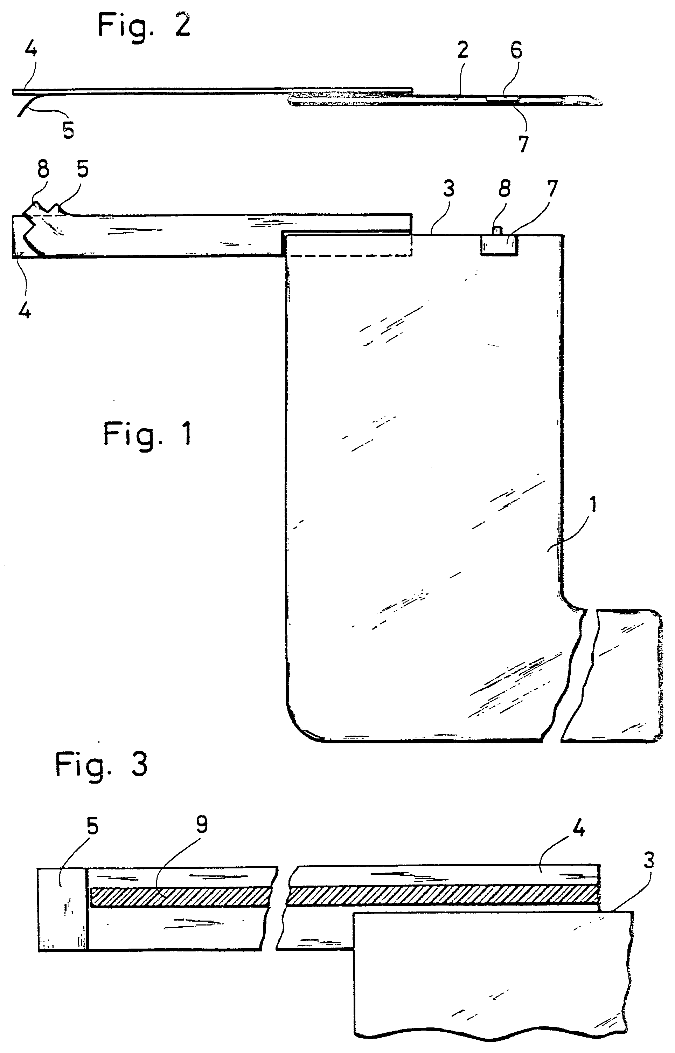

Die Fig. 1 zeigt im Seiten- und die Fig. 2 im Grundriß eine Schutzhülle 1 z. B. in der Konturform eines Beines, die sackartig ausgebildet und mit einer Schlupföffnung 2 versehen ist. Am oberen Rand 3 der Schlupfhülle ist ein Dichtstreifen 4 starr befestigt und mit einer Schutzfolie 5 abgedeckt. Ferner ist im Bereich des oberen Randes ein weiteres Stück Klebstreifen 6 befestigt und mit einer Schutzfolie 7 abgedeckt. Beide Schutzfolien 5 und 7 haben Laschen 8, mit deren Hilfe die Folien abgezogen werden können. Das Lösen der Schutzfolie kann auch über eine an sich bekannte Schnittstelle in der Schutzfolie erfolgen.

Die Fig. 3 zeigt einen Ausschnitt des Haftstreifens 4, auf den oberhalb des Randes 3 der Hülle 1 ein Wulst 9 aus einem weichelastischen Material, z. B. Moosgummi, in einer Stärke von z. B. 1 bis 5 mm aufgebracht ist. Dieser Wulst 9 schafft einen dichten Abschluß auch dann, wenn der Dichtstreifen 4 selbst am Körperteil nicht klebt.

Die Länge des frei abstehenden Streifens und der zugehörigen Schutzfolie 5 ist so bemessen, daß das zu schützende Körperteil umschlossen und die Enden des Klebstreifens sich nach dem dichten Umschließen des Körperteiles überlappen.

Soll die Schutzhülle über einen längeren Zeitraum getragen werden, z. B. im Regen, dann wird die Schutzhülle vorteilhaft aus semipermeablem, atmungsaktivem Material gefertigt, das die Luft durchläßt, gegen Wasser aber dicht abschließt.

**Patentansprüche**

1. Schutzhülle zum flüssigkeitsdichten Abdecken von Körperteilen, insbesondere von Armen und Beinen, unter Verwendung einer sackartigen Kunststoffolie mit einer Schlupföffnung, an der ein bandförmiger elastischer Streifen (4) so befestigt ist, daß er gegen die Schlupfrichtung über den Rand (3) der Hülle (1) übersteht und mit der Innenseites eines frei abstehenden Endes auf seiner Außenseite haftet, dadurch gekennzeichnet, daß der bandförmige, auf der Innenseite mit Klebstoff belegte Dichtstreifen (4) aus zähelastischem Material mit dem einen Ende nur auf einem Teil des Umfanges der Schlupföffnung befestigt und mit dem anderen Ende einen einseitig frei abstehenden Dichtstreifen (4) solcher Länge und solcher Oberfläche bildet, daß der Dichtstreifen (4) nach mehr als einmaligem Umschließen des abzudeckenden Körperteiles unter Einschluß des Randes (3) der Hülle (1) aufeinanderklebend überlappt und daß im Bereich des Schlupfrandes (3) an der Innenseite der Hülle (1) ein Stück Klebstreifen (6) als Haftstelle befestigt ist.

2. Schutzhülle nach Anspruch 1, dadurch gekennzeichnet, daß die Klebeschichten mit Schutzfolien (5, 7) abgedeckt sind und die Schutzfolien mit einer vorzugsweise überstehenden Grifflasche (8) versehen sind.

3. Schutzhülle nach Anspruch 1, dadurch gekennzeichnet, daß oberhalb des Schlupfrandes (3) an der Dichtstreifeninnenseite ein weichelastischer Wulst (9), z. B. aus Moosgummi, aufgebracht ist.

4. Schutzhülle nach Anspruch 1, dadurch gekennzeichnet, daß die Kunststoffolie aus wasserundurchlässigem, atmungsaktivem Material besteht.

**Claims**

1. Fluid-tight protective cover for covering body parts, especially legs and arms, comprising a bag-type plastic foil having a slip opening, to which a elastic band shaped strip (4) is fixed such that it projects opposite to the slip direction over the rim (3) of the covering (1) and sticks with the inner side of its freely projecting end on its outer side, characterized in that the band shaped sealing strip (4) made of viscous-resilient material and provided on the inner side with a layer of adhesive material is fixed with one end only on a part of the circumference of the slip opening and forms with the other end a freely projecting sealing strip (4) of such length and such surface that the sealing strip (4) after surrounding the body part to be covered overlaps in adhesive manner under enclusion of the rim (3) of the cover (1) and that in the area of the rim (3), a strip (6) of adhesive material is fixed to the inner side of the cover (1) for adhesion to that part of the body.

2. A protective covering as in claim 1, characterized in that the adhesive layers are covered by protective film (5, 7), and that said protective film is provided with a gripping tag (8).

3. A protective covering as in claim 1, characterized in that above the rim (3), a strip (9) of soft-resilient material, e. g. of rubber sponge, is applied to the inner side of the sealing strip.

4. A protective covering as in claim 1, characterized in that the plastic foil is a water impermeable but air permeable material.

**Revendications**

1. Enveloppe protectrice pour couvrir en matière étanche des parties d'un corps humain, en particulier des jambes et des bras, comprenant une feuille en plastique en forme d'un sac ayant une ouverture de glissement, à laquelle une bande élastique (4) en forme d'un ruban est fixée, de sorte qu'elle dépasse contre la direction de glissement le bord (3) de l'enveloppe (1) et adhère par la face intérieure de son extrémité dépassant librement à sa face extérieure, caractérisée en ce que la bande d'étanchéité (4) en une matière viscoplastique garnie de colle est fixée avec l'une extrémité seulement à une partie de la périphérie de l'ouverture de glissement et forme avec son autre extrémité une bande d'étanchéité (4) dépassant librement d'un côté et ayant une telle longueur et une telle surface, que la bande d'étanchéité (4) se recouvre en manière collante après plusieurs entourages de la partie du corps à couvrir inclusivement le bord (3) de l'enveloppe (1), et que, dans la zone du bord de glissement (3), une pièce de bande collante (6) est fixée comme position adhésive.

2. Enveloppe protectrice selon la revendication 1, caractérisée en ce que les couches de collage sont couvertes par des feuilles protectrices, et que lesdites couches protectrices sont pourvues d'une languette à poignée (8) étant en préférence débordante.

3. Enveloppe protectrice selon la revendication 1, caractérisée en ce qu'au-dessus du bord de glissement (3), un renflement (9) souple, p. ex. en caoutchouc spongieux, est étendu à la face intérieure de la bande d'étanchéité.

4. Enveloppe protectrice selon la revendication 1, caractérisée en ce que la feuille en plastique se compose d'une matière étanche à l'eau et active à la respiration.

Fig. 2

Fig. 1

Fig. 3